# EUROPÄISCHE PATENTSCHRIFT

(11) **EP 3 639 746 B1**
(45) Veröffentlichungstag und Bekanntmachung des Hinweises auf die Patenterteilung: **21.10.2020**
(21) Anmeldenummer: 19196667.0
(22) Anmeldetag: 11.09.2019
(51) Int. Cl.: A61B 6/00, H05G 1/26, H05G 1/32, A61B 6/03

(54) **BEREITSTELLEN EINES MEDIZINISCHEN BILDDATENSATZES EINES PATIENTEN MITTELS EINER RÖNTGENRÖHRE EINES COMPUTERTOMOGRAPHIESYSTEMS**
PROVISION OF A PATIENT MEDICAL IMAGE DATA SET USING AN X-RAY TUBE OF A COMPUTER TOMOGRAPHY SYSTEM
FOURNITURE D'UN ENSEMBLE DE DONNÉES D'IMAGES MÉDICAL D'UN PATIENT AU MOYEN D'UN TUBE À RAYONS X D'UN SYSTÈME DE TOMODENSITOMÉTRIE

(30) Priorität: 18.10.2018 DE 102018217886
(43) Veröffentlichungstag der Anmeldung: 22.04.2020
(73) Patentinhaber: Siemens Healthcare GmbH, 91052 Erlangen (DE)
(72) Erfinder: Hofmann, Christian, 91052 Erlangen (DE)

(56) Entgegenhaltungen:
- DE-A1-102007 050 889
- DE-A1-102008 034 564
- DE-A1-102012 105 678

## Beschreibung

Die Erfindung betrifft ein Verfahren für ein Bereitstellen eines medizinischen Bilddatensatzes eines Patienten mittels einer Röntgenröhre eines Computertomographiesystems, das Computertomographiesystem und ein zugehöriges Computerprogrammprodukt.

In Abhängigkeit einer klinischen Fragestellung kann eine Messdauer einer bildgebenden Messung variieren. Alternativ oder zusätzlich kann ein Patient die Messdauer der bildgebenden Messung beeinflussen, wenn beispielsweise ein funktioneller Parameter des Patienten zu einem Steuern der bildgebenden Messung verwendet wird. Insbesondere wenn ein interventioneller Eingriff am Patienten während der bildgebenden Messung erfolgt, steht die Messdauer der bildgebenden Messung vor dem Durchführen der bildgebenden Messung typischerweise nicht fest, sondern kann variieren.

Die bildgebende Messung erfolgt typischerweise gemäß einem Messprotokoll, welches insbesondere mehrere, nacheinander abzufahrende Röntgenröhrenstromprofile aufweist. Die Messdauer hängt typischerweise von einer Belastung einer Röntgenröhre gemäß der mehreren Röntgenröhrenstromprofile während der bildgebenden Messung ab. Damit die Belastung während der bildgebenden Messung eine Belastungsgrenze der Röntgenröhre vorzugsweise einhält, wird üblicherweise vor dem Durchführen der bildgebenden Messung eine konservative Abschätzung der Belastung durchgeführt, wodurch die Messdauer der bildgebenden Messung typischerweise im Vergleich zu einer messdaueroptimierten Abschätzung verlängert wird. Denn die konservative Abschätzung umfasst typischerweise längere Ruhezeiten für die Röntgenröhre während der bildgebenden Messung als ein typischer Patient und/oder die klinische Fragestellung üblicherweise erfordern, wobei das Messprotokoll entsprechend angepasst und typischerweise damit die Messdauer verlängert wird. Die Ruhezeiten können beispielsweise einen funktionellen Referenzparameter des typischen Patienten, welcher beispielsweise aus mehreren vorangegangen bildgebenden Messungen gebildet wird, berücksichtigen. Grundsätzlich können die Ruhezeiten derart großzügig gewählt sein, dass die Belastungsgrenze der Röntgenröhre vorzugsweise meistens, besonders vorteilhafterweise immer während der bildgebenden Messung eingehalten wird. Die konservative Abschätzung ermöglicht vorzugsweise, dass die bildgebende Messung durch eine Angabe der Messdauer vor dem Durchführen der bildgebenden Messung planbar wird, weil insbesondere mögliche Variationen in Abhängigkeit von dem Patienten a priori berücksichtigt werden. In diesem Fall werden üblicherweise Belastungsreserven der Röntgenröhre eingeplant, welche die Messdauer verlängern können. Ein Gerät, wie z.B. aus der DE 10 2008 034564 bekannt, kann für ein solches Verfahren verwendet werden.

Der Erfindung liegt die Aufgabe zu Grunde, ein Verfahren für ein Bereitstellen eines medizinischen Bilddatensatzes eines Patienten mittels einer Röntgenröhre eines Computertomographiesystems, ein zugehöriges Computertomographiesystem und ein zugehöriges Computerprogrammprodukt anzugeben, bei welchen eine bildgebende Messung flexibel durchgeführt werden kann.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren für ein Bereitstellen eines medizinischen Bilddatensatzes eines Patienten mittels einer Röntgenröhre eines Computertomographiesystems weist folgende Schritte auf:
- Ermitteln von mehreren Röntgenröhrenstromprofilen der Röntgenröhre in einer Steuereinheit des Computertomographiesystems, wobei die mehreren Röntgenröhrenstromprofile ein erstes Röntgenröhrenstromprofil und ein zweites Röntgenröhrenstromprofil umfassen, ein Messprotokoll für eine bildgebende Messung in dem Computertomographiesystem bilden sowie eine Belastungsgrenze der Röntgenröhre unter Berücksichtigung eines funktionellen Referenzparameters einhalten,
- Erfassen von ersten Rohdaten des Patienten gemäß dem ersten Röntgenröhrenstromprofil mittels der Röntgenröhre, wobei ein funktioneller Parameter des Patienten erfasst wird und wobei zumindest ein Röntgenröhrenstromprofilparameter des ersten Röntgenröhrenstromprofils gemäß dem funktionellen Parameter während des Erfassens der ersten Rohdaten angepasst wird,
- Anpassen des zweiten Röntgenröhrenstromprofils in der Steuereinheit derart, dass das zweite Röntgenröhrenstromprofil in Abhängigkeit von dem zumindest einen angepassten Röntgenröhrenstromprofilparameter die Belastungsgrenze der Röntgenröhre einhält,
- Erfassen von zweiten Rohdaten des Patienten gemäß dem zweiten angepassten Röntgenröhrenstromprofil mittels der Röntgenröhre,
- Rekonstruieren des medizinischen Bilddatensatzes der bildgebenden Messung gemäß den ersten Rohdaten und den zweiten Rohdaten und
- Bereitstellen des medizinischen Bilddatensatzes des Patienten.

Das Verfahren bietet insbesondere folgende Vorteile:
Ein flexibles Durchführen der bildgebenden Messung wird dadurch ermöglicht, dass der zumindest eine angepasste Röntgenröhrenstromprofilparameter für ein Anpassen des zweiten Röntgenröhrenstromprofils verwendet wird. Das Verfahren ermöglicht daher insbesondere ein Anpassen des Messprotokolls, insbesondere der Röntgenröhrenstromprofile, in Abhängigkeit von dem Patienten, vorzugsweise während der bildgebenden Messung. Das Messprotokoll, insbesondere dessen Röntgenröhrenstromprofile, können während der bildgebenden Messung vorteilhafterweise angepasst werden. Durch das flexible Durchführen der bildgebenden Messung kann vorteilhafterweise eine Qualität der ersten Rohdaten und/oder der zweiten Rohdaten, insbesondere eine Bildqualität des medizinischen Bilddatensatzes, erhöht werden.

Vorzugsweise wird eine Messdauer der bildgebenden Messung verringert, weil durch das flexible Durchführen eine konservative Abschätzung, welche typischerweise längere Ruhezeiten als eine messdaueroptimierte Abschätzung einplant, überflüssig und/oder während der bildgebenden Messung analog zur messdaueroptimierten Abschätzung korrigiert wird. Wenn die Messdauer der bildgebenden Messung verringert wird, sind üblicherweise Kosten für das Durchführen der bildgebenden Messung verringert. Alternativ oder zusätzlich kann ein Komfort für den Patienten erhöht sein.

Das Bereitstellen des medizinischen Bilddatensatzes des Patienten mittels der Röntgenröhre des Computertomographiesystems kann insbesondere dann schneller erfolgen, wenn der Patient vorzugsweise regelmäßiger als ein typischer Patient mit dem funktionellen Referenzparameter atmet. Vorzugsweise kann das Durchführen der bildgebenden Messung eher durch die messdaueroptimierte Abschätzung abgebildet sein als durch die konservative Abschätzung.

Der medizinische Bilddatensatz kann beispielsweise einem funktionellen volumetrischen Bilddatensatz entsprechen. Der medizinische Bilddatensatz kann beispielsweise ein erstes medizinisches Bild gemäß den ersten Rohdaten und ein zweites medizinisches Bild gemäß den zweiten Rohdaten aufweisen. Der medizinische Bilddatensatz kann insbesondere in einem DICOM-Bildformat vorliegen.

Wenn beispielsweise eine klinische Fragestellung eine Herzuntersuchung des Patienten betrifft, kann der medizinische Bilddatensatz vorzugsweise für die Herzuntersuchung verwendet werden. In anderen Worten wird typischerweise kein weiterer medizinischer Bilddatensatz für die klinische Fragestellung bereitgestellt, weil der medizinische Bilddatensatz typischerweise dem funktionellen volumetrischen Bilddatensatz entsprechen kann. Die klinische Fragestellung kann insbesondere eine Untersuchung einer physiologischen Funktion des Patienten, beispielsweise die Herzuntersuchung und/oder eine Atemuntersuchung betreffen. Grundsätzlich ist es denkbar, dass nach dem Bereitstellen des medizinischen Bilddatensatzes gemäß einer ersten klinischen Fragestellung der weitere medizinische Bilddatensatz gemäß einer zweiten klinischen Fragestellung bereitgestellt wird.

Im Allgemeinen umfasst ein Röntgenröhrenstromprofil der Röntgenröhre zumindest einen Röntgenröhrenstromprofilparameter, beispielsweise eine Röntgenröhrenstromdauer, währenddessen üblicherweise von der Röntgenröhre eine Röntgenstrahlung mit einer Röntgenröhrenstromamplitude emittiert wird. Die Röntgenröhrenstromamplitude kann während der Röntgenröhrenstromdauer konstant sein oder variieren. Zusätzlich kann das Röntgenröhrenstromprofil eine Röntgenröhrenruhezeit umfassen, währenddessen typischerweise keine Röntgenstrahlung emittiert wird und/oder die Röntgenröhre abkühlen kann. Ein Abfahren des Röntgenröhrenstromprofils umfasst insbesondere das Emittieren der Röntgenstrahlung mit der Röntgenröhrenstromamplitude während der Röntgenröhrenstromdauer. Das Abfahren des Röntgenröhrenstromprofils kann zusätzlich ein Einhalten der Röntgenröhrenruhezeit umfassen. Eine Belastung der Röntgenröhre korreliert üblicherweise mit einem Produkt aus der Röntgenröhrenstromdauer und der Röntgenröhrenstromamplitude. Von der Belastung der Röntgenröhre hängt typischerweise eine Dosisbelastung des Patienten ab. Die Röntgenröhre wird insbesondere während der Röntgenröhrenstromdauer belastet.

Die mehreren Röntgenröhrenstromprofile bilden insofern das Messprotokoll für die bildgebende Messung, weil durch das Abfahren der mehreren Röntgenstromprofile üblicherweise diejenigen Rohdaten erfasst werden, welche für das Bereitstellen des medizinischen Bilddatensatzes verwendet werden. Nach dem Abfahren der mehreren Röntgenröhrenstromprofile ist üblicherweise das Erfassen der Rohdaten der medizinischen Bildgebung, insbesondere gemäß dem Messprotokoll, beendet. Typischerweise wird das erste Röntgenröhrenstromprofil vor dem zweiten Röntgenröhrenstromprofil abgefahren. Ein Beginn des ersten Röntgenröhrenstromprofils und ein Ende des zweiten Röntgenröhrenstromprofils bilden insbesondere eine Messdauer der bildgebenden Messung. Das Messprotokoll weist typischerweise zusätzlich zu den mehreren Röntgenröhrenstromprofilen einen Tischvorschub, eine Röntgenröhrenspannung, den Messbereich, einen Rekonstruktionskern und/oder eine Röntgenstrahlenkollimation auf. Das Messprotokoll kann beispielsweise durch einen Nutzer zumindest teilweise festgelegt und/oder angepasst werden. Das Computertomographiesystem kann eine Planungseinheit für das Festlegen und/oder Anpassen des Messprotokolls durch den Nutzer umfassen. Die Planungseinheit kann eine Anzeigeeinheit und/oder Eingabemittel aufweisen.

Die Belastungsgrenze der Röntgenröhre kann beispielsweise von einem Hersteller der Röntgenröhre und/oder des Computertomographiesystems festgelegt und/oder unveränderlich sein. Die Belastungsgrenze der Röntgenröhre wird vorzugsweise derart eingehalten, dass die Belastung der Röntgenröhre die Belastungsgrenze während der bildgebenden Messung, insbesondere während dem Abfahren der Röntgenröhrenstromprofile, nicht übersteigt, sondern vorzugsweise einhält. Die Röntgenröhre wird vorzugsweise während der bildgebenden Messung im Normbereich betrieben, wenn die Belastungsgrenze der Röntgenröhre eingehalten wird. Wenn die Röntgenröhre beispielsweise außerhalb des Normbereichs betrieben wird, kann die Belastung der Röntgenröhre ein unplanmäßiges Abbrechen der medizinischen Bildgebung ohne Bereitstellen des medizinischen Bildes zur Folge haben und/oder einen verstärkten Verschleiß der Röntgenröhre und/oder eine Zerstörung der Röntgenröhre bewirken und/oder eine zusätzliche Abkühlphase nach der medizinischen Bildgebung nötig machen. Ob die Belastungsgrenze während der bildgebenden Messung eingehalten wird, kann beispielsweise mittels eines Sensors des Computertomographiesystems ermittelt und an die Steuereinheit übertragen werden, wobei die Steuereinheit beispielsweise im Betrieb außerhalb des Normbereichs die medizinische Bildgebung abbricht und/oder die zusätzliche Abkühlphase, insbesondere ein Verlängern einer Röntgenröhrenruhezeit, festlegt. Vorteilhafterweise kann durch das Einhalten der Belastungsgrenze eine Lebensdauer der Röntgenröhre erhöht und/oder ein Wartungsaufwand verringert werden.

Die Steuereinheit ist vorzugsweise zu einem Steuern der bildgebenden Messung, insbesondere gemäß dem funktionellen Parameter des Patienten, ausgebildet. Die Steuereinheit ist vorzugsweise zum derartigen Ermitteln der mehreren Röntgenröhrenstromprofile der Röntgenröhre ausgebildet, dass insbesondere die Belastungsgrenze eingehalten wird. Die Steuereinheit ermittelt die mehreren Röntgenröhrenstromprofile typischerweise nach der messdaueroptimierten Abschätzung, weil das zweite Röntgenröhrenstromprofil üblicherweise angepasst wird, wenn der zumindest eine Röntgenröhrenstromprofilparameter des ersten Röntgenröhrenstromprofils angepasst wird. Grundsätzlich ist es denkbar, dass die Steuereinheit die mehreren Röntgenröhrenstromprofil gemäß einer konservativen Abschätzung ermittelt. Die messdaueroptimierte Abschätzung kann typischerweise in Hinblick auf die Messdauer der bildgebenden Messung vorteilhaft sein, insbesondere wenn die Messdauer dadurch verkürzt wird. Die konservative Abschätzung berücksichtigt typischerweise mehr Variationen, welche bei der medizinischen Bildgebung auftreten können, als die messdaueroptimierte Abschätzung. Die Röntgenröhrenruhezeit ist bei der konservativen Abschätzung üblicherweise länger als bei der messdaueroptimierten Abschätzung.

Für das Ermitteln der mehreren Röntgenröhrenstromprofile kann die Steuereinheit Steuerprogrammcodemittel aufweisen, in welchen die Belastungsgrenze vorzugsweise abgebildet ist. Eine Recheneinheit des Computertomographiesystems ist insbesondere für eine Ausführung der Steuerprogrammcodemittel ausgebildet. Die Recheneinheit kann insbesondere einen Arbeitsspeicher und/oder einen Prozessor und/oder eine Speichereinheit aufweisen. Die Belastungsgrenze kann typischerweise aus der Speichereinheit abgerufen werden.

Der funktionelle Referenzparameter wird beispielsweise derart ermittelt, dass ein Referenzwert aus einem Fachbuch und/oder gemäß einem typischen Patienten aus einem Patientenkollektiv bestimmt wird. Grundsätzlich ist es denkbar, dass der funktionelle Referenzparameter vor der bildgebenden Messung in Abhängigkeit von dem Patienten erfasst wird und der funktionelle Parameter des Patienten während der bildgebenden Messung erfasst wird. In diesem Fall unterscheiden sich der funktionelle Referenzparameter und der funktionelle Parameter des Patienten insbesondere in einem Zeitpunkt des Erfassens. Der funktionelle Parameter des Patienten kann beispielsweise einem funktionellen Echtzeitparameter des Patienten während der bildgebenden Messung entsprechen. Der funktionelle Referenzparameter kann einen Toleranzbereich berücksichtigen. Der funktionelle Referenzparameter berücksichtigt insbesondere eine Regularität und/oder eine Amplitude und/oder eine Frequenz der physiologischen Funktion des Patienten. Die physiologische Funktion kann insbesondere die Atmung des Patienten und/oder die Herzphase des Patienten umfassen.

Das Einhalten der Belastungsgrenze kann dem Berücksichtigen der Belastungsgrenze entsprechen. Das Einhalten der Belastungsgrenze kann umfassen, dass eine Gesamtbelastungsgrenze für die bildgebende Messung festgelegt wird, welche die mehreren Röntgenröhrenstromprofile einhalten müssen. Alternativ oder zusätzlich kann das Einhalten der Belastungsgrenze umfassen, dass jedes Röntgenröhrenstromprofil eine Profilbelastungsgrenze einhält. Typischerweise werden die mehreren Röntgenröhrenstromprofile derart ermittelt, dass die mehreren Röntgenröhrenstromprofile die Profilbelastungsgrenze und die Gesamtbelastungsgrenze einhalten. Die Gesamtbelastungsgrenze wird beispielsweise eingehalten, wenn eine Summe der Belastungen durch die mehreren Röntgenröhrenstromprofile kleiner oder gleich der Gesamtbelastungsgrenze ist.

Die Belastungsgrenze kann zusätzlich zu dem funktionellen Referenzparameter eine maximale Röntgenröhrenstromamplitude und/oder eine maximale Röntgenröhrenstromdauer und/oder eine maximale Belastung der Röntgenröhre und/oder eine maximale Dosisbelastung des Patienten berücksichtigen. Die Belastungsgrenze kann von aggregierten Betriebsstunden der Röntgenröhre abhängen. Die Betriebsstunden werden insbesondere von der Messdauer der bildgebenden Messung und von Messdauern weiterer bildgebender Messungen ermittelt. Die Betriebsstunden werden typischerweise von Röntgenröhrenstromprofilen der Röntgenröhre, beispielsweise seit einer Herstellung der Röntgenröhre und/oder seit einer Wartung der Röntgenröhre abhängen. Typischerweise kann eine Röntgenröhre desto höher belastet werden, je weniger Betriebsstunden die Röntgenröhre aufweist. Beispielsweise weist die Röntgenröhre und/oder das Computertomographiesystem die Speichereinheit für ein Abspeichern der Betriebsstunden der Röntgenröhre auf. Die Belastungsgrenze kann in Abhängigkeit von der messdaueroptimierten Abschätzung niedriger sein als im Vergleich zu einer Abhängigkeit von der konservativen Abschätzung. Die messdaueroptimierte Abschätzung kann üblicherweise zu einer stärkeren Belastung der Röntgenröhre führen als die konservative Abschätzung. Grundsätzlich ist die Röntgenröhrenruhezeit desto länger, je niedriger die Belastungsgrenze der Röntgenröhre ist.

Das Messprotokoll kann eine erste funktionelle Phase und eine zweite funktionelle Phase umfassen, wobei vorzugsweise die ersten Rohdaten während der ersten funktionellen Phase und die zweiten Rohdaten während der zweiten funktionellen Phase erfasst werden. Die erste funktionelle Phase und die zweite funktionelle Phase können sich vorzugsweise in einer Phase innerhalb einer Periode der physiologischen Funktion des Patienten unterscheiden, wobei beispielsweise die ersten Rohdaten während eines Einatmens des Patienten und die zweiten Rohdaten während eines Ausatmens des Patienten erfassen werden. Alternativ oder zusätzlich kann die Periode der physiologischen Funktion eine Kontrastmittelanreicherung und eine Kontrastmittelauswaschung und/oder Herzphasen eines Herzrhythmus des Patienten umfassen. Die Kontrastmittelanreicherung und die Kontrastmittelauswaschung sind insbesondere Kontrastmittelphasen. Die erste funktionelle Phase und die zweite funktionelle Phase können sich in einer z-Position eines Messbereichs der bildgebenden Messung derart unterscheiden, dass die ersten Rohdaten während der ersten funktionellen Phase an einer ersten z-Position und die zweiten Rohdaten während der zweiten funktionellen Phase an einer zweiten z-Position erfasst werden. In diesem Fall kann beispielsweise das Einatmen, insbesondere eines ersten Atemzyklus, des Patienten an der ersten z-Position und das Einatmen, insbesondere eines zweiten Atemzyklus, an der zweiten z-Position erfasst werden, wobei der erste Atemzyklus typischerweise vor dem zweiten Atemzyklus auftritt. Grundsätzlich ist es denkbar, dass die erste funktionelle Phase und die zweite funktionelle Phase sich in der Phase innerhalb der Periode der physiologischen Funktion des Patienten und in der z-Position unterscheiden. Beispielsweise kann in diesem Fall das Einatmen des Patienten an der ersten z-Position und das Ausatmen an der zweiten z-Position erfasst werden. Die mehreren Röntgenröhrenstromprofile können beispielsweise in Abhängigkeit von der z-Position des Messbereichs der bildgebenden Messung und/oder von der funktionellen Phase des Messprotokolls variieren und/oder ermittelt werden.

Die Steuereinheit kann typischerweise gemäß dem ersten Röntgenröhrenstromprofil das Erfassen der ersten Rohdaten und/oder gemäß dem zweiten Röntgenröhrenstromprofil das Erfassen der zweiten Rohdaten auslösen. Das erste Röntgenröhrenstromprofil wird üblicherweise für das Erfassen der ersten Rohdaten abgefahren. Das zweite Röntgenröhrenstromprofil wird üblicherweise für das Erfassen der zweiten Rohdaten abgefahren.

Während dem Abfahren des Röntgenröhrenstromprofils wird typischerweise der funktionelle Parameter des Patienten vorzugsweise gemäß einer Abtastrate erfasst. Die Abtastrate beträgt beispielsweise mehr als 1 Hz, vorteilhafterweise 50 Hz und/oder besonders vorteilhafterweise weniger als 50 Hz. Die Abtastrate beträgt vorteilhafterweise zwischen 0,1 und 200 Hz, besonders vorteilhafterweise zwischen 40 und 60 Hz.

Der funktionelle Parameter kann beispielsweise mittels einer funktionellen Patientenüberwachungseinheit erfasst werden, welche insbesondere einen Atemgurt, ein Spirometer, eine Kamera und/oder einen Echokardiographen umfasst. Die Kamera kann insbesondere eine 3D-Kamera und/oder eine Video-Kamera und/oder Infrarot-Kamera sein.

Durch das Anpassen des zumindest einen Röntgenröhrenstromprofilparameters wird das erste Röntgenröhrenstromprofil typischerweise mit dem funktionellen Parameter, insbesondere mit der physiologischen Funktion des Patienten, synchronisiert. Das Anpassen des zumindest einen Röntgenröhrenstromprofilparameters erfolgt üblicherweise, wenn der funktionelle Referenzparameter und der funktionelle Parameter des Patienten sich unterscheiden, insbesondere in der Regularität und/oder in der Amplitude und/oder in der Frequenz der physiologischen Funktion des Patienten. Typischerweise können der funktionelle Referenzparameter und der funktionelle Parameter des Patienten derselben klinischen Fragestellung zugeordnet werden.

Das Anpassen des zumindest einen Röntgenröhrenstromprofilparameters umfasst insbesondere ein Anpassen der bildgebenden Messung, beispielsweise auf Basis der konservativen Abschätzung und/oder messdaueroptimierten Abschätzung, an den Patienten. Das Anpassen des zumindest einen Röntgenröhrenstromprofilparameters erfolgt vorzugsweise mit einer Anpassfrequenz höher als 1 Hz, vorteilhafterweise höher als 50 Hz, besonders vorteilhafterweise höher als 200 Hz. Die Anpassfrequenz beträgt vorteilhafterweise zwischen 0,1 und 500 Hz, besonders vorteilhafterweise zwischen 10 und 100 Hz. Die Anpassfrequenz kann der Frequenz der funktionellen Patientenüberwachungseinheit entsprechen. Vorzugsweise kann die Steuereinheit gemäß der Anpassfrequenz das erste Röntgenröhrenstromprofil, insbesondere den zumindest einen Röntgenröhrenstromprofilparameter, an den funktionellen Parameter des Patienten anpassen.

Das Anpassen des zumindest einen Röntgenröhrenstromprofilparameters erfolgt insbesondere derart, dass ein Integral des ersten Röntgenröhrenstromprofils beim Anpassen verkleinert oder vergrößert wird oder konstant bleibt. Das Integral des ersten Röntgenröhrenstromprofils entspricht typischerweise einer Fläche gemäß der ersten Röntgenröhrenstromamplitude und der ersten Röntgenröhrenstromdauer. Typischerweise korreliert das Integral mit der Belastung der Röntgenröhre. Beim Anpassen des zumindest einen Röntgenröhrenstromprofilparameters wird typischerweise die Belastungsgrenze der Röntgenröhre berücksichtigt. Das Anpassen des zumindest einen Röntgenröhrenstromprofilparameters kann ein Verschieben und/oder Ändern des zumindest einen Röntgenröhrenstromprofilparameters umfassen.

Die Steuereinheit kann beispielsweise die Röntgenröhre derart steuern, dass die ersten Rohdaten während der ersten funktionellen Phase und vor der zweiten funktionellen Phase erfasst werden. Damit die ersten Rohdaten während der ersten funktionellen Phase und vor der zweiten funktionellen Phase erfasst werden können, wird beispielsweise der zumindest eine Röntgenröhrenstromprofilparameter angepasst. Die Steuereinheit kann vorzugsweise ermitteln, ob die ersten Rohdaten während der ersten funktionellen Phase und vor der zweiten funktionellen Phase erfasst werden. Die Steuereinheit kann insbesondere durch ein Vergleichen des funktionellen Parameters mit dem Messprotokoll ermitteln und das Erfassen der ersten Rohdaten derart steuern, dass die ersten Rohdaten während der ersten funktionellen Phase und vor der zweiten funktionellen Phase erfasst werden.

Nach dem Erfassen der ersten Rohdaten und/oder vor dem Erfassen der zweiten Rohdaten wird üblicherweise das zweite Röntgenröhrenstromprofil in der Steuereinheit angepasst.

Das Anpassen des zweiten Röntgenröhrenstromprofils kann derart erfolgen, dass ein Integral des zweiten Röntgenröhrenstromprofils beim Anpassen verkleinert oder vergrößert wird oder konstant bleibt. Wenn das Integral des zweiten Röntgenröhrenstromprofils konstant bleibt, werden üblicherweise zwei Röntgenröhrenstromprofilparameter angepasst, insbesondere derart, dass das Anpassen der zwei Röntgenröhrenstromprofilparameter sich in Hinblick auf das Integral des zweiten Röntgenröhrenstromprofils kompensieren. Das Anpassen kann ein Erhöhen, ein Verlängern, ein Verkürzen und/oder ein Verringern des zweiten Röntgenröhrenstromprofils umfassen.

Dass das zweite Röntgenröhrenstromprofil in Abhängigkeit von dem zumindest einen angepassten Röntgenröhrenstromprofilparameter die Belastungsgrenze der Röntgenröhre einhält, bedeutet insbesondere, dass der zumindest eine angepasste Röntgenröhrenstromprofilparameter und die Belastungsgrenze der Röntgenröhre beim Anpassen als Eingangsparameter eingehen. Das Anpassen des zweiten Röntgenröhrenstromprofilparameters kann gemäß der konservativen Abschätzung und/oder der messdaueroptimierten Abschätzung erfolgen.

Beim Erfassen der zweiten Rohdaten kann grundsätzlich der funktionelle Parameter des Patienten erfasst werden und zumindest ein Röntgenröhrenstromprofilparameter des zweiten Röntgenröhrenstromprofils angepasst werden. Die Steuereinheit kann beispielsweise die Röntgenröhre derart steuern, dass die zweiten Rohdaten nach der ersten funktionellen Phase und während der zweiten funktionellen Phase erfasst werden.

Die ersten Rohdaten und/oder die zweiten Rohdaten können insbesondere Projektionsdaten des Computertomographiesystems sein. Die ersten Rohdaten und/oder die zweiten Rohdaten können beispielsweise in einem Radiologieinformationssystem und/oder in einem PACS-Bildarchivierungssystem und/oder in der Speichereinheit des Computertomographiesystems gespeichert werden. Die Steuereinheit kann eine Rekonstruktionseinheit für das Rekonstruieren des medizinischen Bilddatensatzes umfassen. Die Steuereinheit ist typischerweise mit dem Radiologieinformationssystem und/oder dem PACS-Bildarchivierungssystem und/oder der Speichereinheit verbunden, insbesondere für das Abrufen der ersten Rohdaten und/oder der zweiten Rohdaten und/oder für das Bereitstellen des medizinischen Bilddatensatzes. Das Rekonstruieren erfolgt insbesondere gemäß dem Rekonstruktionskern des Messprotokolls. Das Rekonstruieren umfasst insbesondere eine gefilterte Rückprojektion und/oder eine iterative Rekonstruktionsvorschrift. Der medizinische Bilddatensatz bildet insbesondere den Messbereich, beispielsweise mit den mehreren z-Positionen und/oder in der funktionellen Phase des Messprotokolls, ab.

Der medizinische Bilddatensatz kann insbesondere auf der Anzeigeeinheit des Computertomographiesystems bereitgestellt werden. Beispielsweise kann der Nutzer mittels des auf der Anzeigeeinheit bereitgestellten medizinischen Bilddatensatzes die klinische Fragestellung der medizinischen Bildgebung evaluieren. Der medizinische Bilddatensatz kann vor und/oder nach dem Bereitstellen in das Radiologieinformationssystem und/oder das PACS-Bildarchivierungssystem und/oder die Speichereinheit übertragen werden.

Eine Ausführungsform sieht vor, dass der funktionelle Parameter des Patienten eine Atmung und/oder eine Herzphase des Patienten beschreibt. Diese Ausführungsform ist insbesondere vorteilhaft, weil die klinische Fragestellung, welche die Atemuntersuchung und/oder die Herzuntersuchung betrifft, vorteilhafterweise mittels des medizinischen Bildes evaluiert werden kann.

Eine Ausführungsform sieht vor, dass sich das Messprotokoll für das Erfassen der ersten Rohdaten und das Messprotokoll für das Erfassen der zweiten Rohdaten in zumindest einem Parameter der folgenden Liste unterscheiden:
- in der z-Position des Messbereichs des Messprotokolls,
- in der Phase innerhalb der Periode der physiologischen Funktion des Patienten. In dieser Ausführungsform weist das Messprotokoll typischerweise die erste funktionelle Phase und die zweite funktionelle Phase auf. Wenn sich die Messprotokolle in der z-Position des Messbereichs unterscheiden, entspricht der medizinische Bilddatensatz insbesondere einem volumetrischen Bilddatensatz. Wenn sich die Messprotokolle in der Phase innerhalb der Periode der physiologischen Funktion des Patienten unterscheiden, entspricht der medizinische Bilddatensatz insbesondere einem funktionellen Bilddatensatz. Wenn sich die Messprotokolle in der z-Position und in der Phase innerhalb der Periode der physiologischen Funktion des Patienten unterscheiden, entspricht der medizinische Bilddatensatz insbesondere dem funktionellen volumetrischen Bilddatensatz, welcher beispielsweise ein 4D-Bilddatensatz ist.

Eine Ausführungsform sieht vor, dass das Anpassen des zweiten Röntgenröhrenstromprofils unter Verwendung eines künstlichen neuronalen Netzes erfolgt. Diese Ausführungsform ist insofern vorteilhaft, weil das künstliche neuronale Netz ein besseres Anpassen des zweiten Röntgenröhrenstromprofils ermöglichen kann, weil das künstliche neuronale Netz typischerweise vor der bildgebenden Messung trainiert und/oder während der bildgebenden Messung weitergebildet wird. Das künstliche neuronale Netz weist typischerweise eine Eingangsschicht und eine Ausgangsschicht auf, welche typischerweise über Kanten zwischen der Eingangsschicht und der Ausgangsschicht und/oder über Kanten zwischen zumindest einer versteckten Schicht zwischen der Eingangsschicht und der Ausgangsschicht verbunden werden. Beim Trainieren und/oder beim Weiterbilden werden üblicherweise Gewichte der Kanten ermittelt, beispielsweise gemäß vorherigen bildgebenden Messungen und/oder gemäß der bildgebenden Messung.

Eine Ausführungsform sieht vor, dass die Belastungsgrenze der Röntgenröhre einen Temperaturschwellwert der Röntgenröhre umfasst. Der Temperaturschwellwert beschreibt insbesondere eine Maximaltemperatur der Röntgenröhre. Im Normbetrieb kann die Röntgenröhre typischerweise bis zur Maximaltemperatur während der bildgebenden Messung belastet werden. Der Sensor des Computertomographiesystems kann insbesondere ein Temperatursensor sein. Diese Ausführungsform ist vorteilhaft, weil sie verhindert, dass aufgrund eines Überschreitens der Maximaltemperatur der Verschleiß der Röntgenröhre insbesondere exponentiell zunimmt und/oder die Röntgenröhren zerstört wird. Diese Ausführungsform ermöglicht insbesondere ein längeres Betreiben der Röntgenröhre.

Eine Ausführungsform sieht vor, dass der zumindest eine Röntgenröhrenstromprofilparameter eine erste Röntgenröhrenstromamplitude und/oder eine erste Röntgenröhrenstromdauer und/oder eine erste Röntgenröhrenruhezeit umfasst. Diese Ausführungsform umfasst vorteilhafterweise, dass gemäß dem funktionellen Parameter während des Erfassens der ersten Rohdaten die erste Röntgenröhrenstromamplitude und/oder die erste Röntgenröhrenstromdauer und/oder die erste Röntgenröhrenruhezeit angepasst wird. Es ist grundsätzlich denkbar, dass beim Anpassen des zumindest einen Röntgenröhrenstromprofilparameters eine beliebige Kombination der ersten Röntgenröhrenstromamplitude, der ersten Röntgenröhrenstromdauer und der ersten Röntgenröhrenruhezeit angepasst wird. Das Anpassen kann ein Erhöhen, ein Verlängern, ein Verkürzen und/oder ein Verringern des zumindest einen Röntgenröhrenstromprofilparameters umfassen.

Eine Ausführungsform sieht vor, dass das Anpassen des zweiten Röntgenröhrenstromprofils ein Anpassen einer zweiten Röntgenröhrenstromamplitude und/oder einer zweiten Röntgenröhrenstromdauer und/oder einer zweiten Röntgenröhrenruhezeit umfasst. Diese Ausführungsform präzisiert vorteilhafterweise, dass beim Anpassen des zweiten Röntgenröhrenstromprofils lediglich ein einziger Röntgenröhrenstromprofilparameter oder mehrere Röntgenröhrenröhrenstromprofilparameter angepasst werden können.

Eine Ausführungsform sieht vor, dass in Abhängigkeit von einer Vergrößerung des ersten Röntgenröhrenstromprofils durch den funktionellen Parameter des Patienten eine Verkleinerung des zweiten Röntgenröhrenstromprofils zur Einhaltung der Belastungsgrenze der Röntgenröhre beim Anpassen erfolgt. Die Vergrößerung des ersten Röntgenröhrenstromprofils umfasst insbesondere, dass das Integral des ersten Röntgenröhrenstromprofils erhöht wird. Dass das erste Röntgenröhrenstromprofil und/oder das zweite Röntgenröhrenstromprofil durch den funktionellen Parameter verkleinert und/oder vergrößert wird, bedeutet insbesondere, dass die mehreren Röntgenröhrenstromprofile gemäß dem funktionellen Parameter des Patienten, vorzugsweise laufend während der bildgebenden Messung an die physiologische Funktion des Patienten, angepasst werden.

In diesem Ausführungsbeispiel wird vorteilhafterweise eine Anpassung des ersten Röntgenröhrenstromprofils durch das Anpassen des zumindest einen Röntgenröhrenstromprofilparameters dadurch kompensiert, dass das zweite Röntgenröhrenstromprofil angepasst wird.

Eine Ausführungsform sieht vor, dass die zweite Röntgenröhrenstromamplitude verringert wird. Diese Ausführungsform beschreibt vorteilhafterweise, dass das Anpassen des Röntgenröhrenstromprofils zu einer Verringerung der Dosisbelastung des Patienten führen kann.

Eine Ausführungsform sieht vor, dass die zweite Röntgenröhrenstromdauer verringert wird. In dieser Ausführungsform ist vorzugsweise die Messdauer verkürzt.

Eine Ausführungsform sieht vor, dass in Abhängigkeit von einer Vergrößerung des ersten Röntgenröhrenstromprofils durch den funktionellen Parameter des Patienten eine Verlängerung der zweiten Röntgenröhrenruhezeit erfolgt. Diese Ausführungsform ist insbesondere vorteilhaft, weil durch das Anpassen der zweiten Röntgenröhrenruhezeit die Dosisbelastung des Patienten durch das zweite Röntgenröhrenstromprofil konstant bleibt.

Eine Ausführungsform sieht vor, dass in Abhängigkeit von einer Verkleinerung des ersten Röntgenröhrenstromprofils durch den funktionellen Parameter des Patienten eine Vergrößerung des zweiten Röntgenröhrenstromprofils beim Anpassen erfolgt. Dieses Ausführungsbeispiel umfasst insbesondere einen Vorteil, dass die Belastung der Röntgenröhre insbesondere zur Einhaltung der Gesamtbelastungsgrenze auf die mehreren Röntgenröhrenstromprofile verteilt wird.

Eine Ausführungsform sieht vor, dass die zweite Röntgenröhrenstromamplitude und/oder die zweite Röntgenröhrenstromdauer vergrößert werden. Durch das Vergrößern des Integrals des zweiten Röntgenröhrenstromprofils kann vorteilhafterweise die Qualität der zweiten Rohdaten, insbesondere die Bildqualität des medizinischen Bilddatensatzes erhöht werden.

Das Computertomographiesystem weist die Steuereinheit und die Röntgenröhre auf und ist nach dem Verfahren für das Bereitstellen des medizinischen Bilddatensatzes mittels der Röntgenröhre ausgebildet.

Ein Computerprogrammprodukt, welches direkt in einen Speicher der Recheneinheit ladbar ist, weist Programmcodemittel auf, um das Verfahren für das Bereitstellen des medizinischen Bilddatensatzes mittels der Röntgenröhre des Computertomographiesystems auszuführen, wenn das Computerprogrammprodukt in der Recheneinheit ausgeführt wird. Der Speicher kann die Speichereinheit und/oder den Arbeitsspeicher aufweisen.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Verfahren für ein Bereitstellen eines medizinischen Bilddatensatzes eines Patienten mittels einer Röntgenröhre eines Computertomographiesystems in einem ersten Ausführungsbeispiel,
Fig. 2 das Verfahren in einem zweiten Ausführungsbeispiel,
Fig. 3 schematisch die mehreren Röntgenröhrenstromprofile vor einem Erfassen von ersten Rohdaten,
Fig. 4 schematisch die mehreren Röntgenröhrenstromprofile nach dem Erfassen der ersten Rohdaten und vor einem Anpassen des zweiten Röntgenröhrenstromprofils,
Fig. 5 schematisch die mehreren Röntgenröhrenstromprofile nach dem Anpassen des zweiten Röntgenröhrenstromprofils und
Fig. 6 ein Computertomographiesystem.

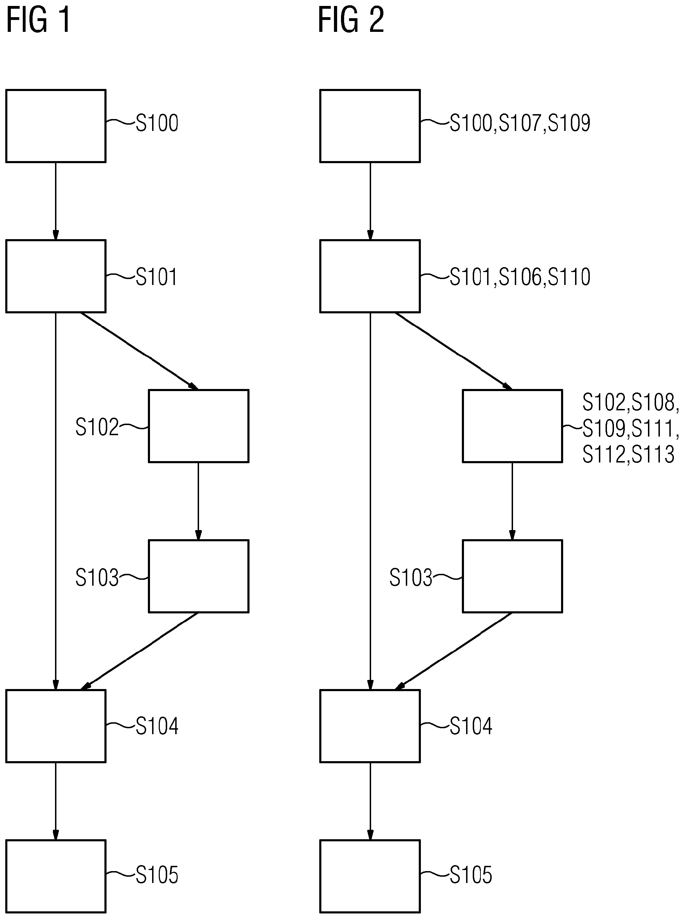

**Fig. 1** zeigt ein Flussdiagramm des Verfahrens für das Bereitstellen des medizinischen Bilddatensatzes des Patienten mittels der Röntgenröhre des Computertomographiesystems in dem ersten Ausführungsbeispiel.

Verfahrensschritt S100 kennzeichnet ein Ermitteln von mehreren Röntgenröhrenstromprofilen der Röntgenröhre in einer Steuereinheit des Computertomographiesystems, wobei die mehreren Röntgenröhrenstromprofile ein erstes Röntgenröhrenstromprofil und ein zweites Röntgenröhrenstromprofil umfassen, ein Messprotokoll für eine bildgebende Messung in dem Computertomographiesystem bilden sowie eine Belastungsgrenze der Röntgenröhre unter Berücksichtigung eines funktionellen Referenzparameters einhalten.

Verfahrensschritt S101 kennzeichnet ein Erfassen von ersten Rohdaten des Patienten gemäß dem ersten Röntgenröhrenstromprofil mittels der Röntgenröhre, wobei ein funktioneller Parameter des Patienten erfasst wird und wobei zumindest ein Röntgenröhrenstromprofilparameter des ersten Röntgenröhrenstromprofils gemäß dem funktionellen Parameter während des Erfassens der ersten Rohdaten angepasst wird.

Verfahrensschritt S102 kennzeichnet ein Anpassen des zweiten Röntgenröhrenstromprofils in der Steuereinheit derart, dass das zweite Röntgenröhrenstromprofil in Abhängigkeit von dem zumindest einen angepassten Röntgenröhrenstromprofilparameter die Belastungsgrenze der Röntgenröhre einhält.

Verfahrensschritt S103 kennzeichnet ein Erfassen von zweiten Rohdaten des Patienten gemäß dem zweiten angepassten Röntgenröhrenstromprofil mittels der Röntgenröhre.

Verfahrensschritt S104 kennzeichnet ein Rekonstruieren des medizinischen Bilddatensatzes der bildgebenden Messung gemäß den ersten Rohdaten und den zweiten Rohdaten.

Verfahrensschritt S105 kennzeichnet ein Bereitstellen des medizinischen Bilddatensatzes des Patienten.

**Fig. 2** zeigt ein Flussdiagramm des Verfahrens in einem zweiten Ausführungsbeispiel.

Verfahrensschritt S106 kennzeichnet, dass der funktionelle Parameter des Patienten eine Atmung und/oder eine Herzphase des Patienten beschreibt.

Verfahrensschritt S107 kennzeichnet, dass sich das Messprotokoll für das Erfassen der ersten Rohdaten und das Messprotokoll für das Erfassen der zweiten Rohdaten in zumindest einem Parameter der folgenden Liste unterscheiden:
- in einer z-Position eines Messbereichs des Messprotokolls,
- in einer Phase innerhalb einer Periode einer physiologischen Funktion des Patienten.

Verfahrensschritt S108 kennzeichnet, dass das Anpassen des zweiten Röntgenröhrenstromprofils unter Verwendung eines künstlichen neuronalen Netzes erfolgt.

Verfahrensschritt S109 kennzeichnet, dass die Belastungsgrenze der Röntgenröhre einen Temperaturschwellwert der Röntgenröhre umfasst.

Verfahrensschritt S110 kennzeichnet, dass der zumindest eine Röntgenröhrenstromprofilparameter eine erste Röntgenröhrenstromamplitude und/oder eine erste Röntgenröhrenstromdauer und/oder eine erste Röntgenröhrenruhezeit umfasst.

Verfahrensschritt S111 kennzeichnet, dass das Anpassen des zweiten Röntgenröhrenstromprofils ein Anpassen einer zweiten Röntgenröhrenstromamplitude und/oder einer zweiten Röntgenröhrenstromdauer und/oder einer zweiten Röntgenröhrenruhezeit umfasst.

Verfahrensschritt S112 kennzeichnet, dass in Abhängigkeit von einer Vergrößerung des ersten Röntgenröhrenstromprofils durch den funktionellen Parameter des Patienten eine Verkleinerung des zweiten Röntgenröhrenstromprofils zur Einhaltung der Belastungsgrenze der Röntgenröhre beim Anpassen erfolgt.

Verfahrensschritt S113 kennzeichnet, dass in Abhängigkeit von einer Verkleinerung des ersten Röntgenröhrenstromprofils durch den funktionellen Parameter des Patienten eine Vergrößerung des zweiten Röntgenröhrenstromprofils beim Anpassen erfolgt.

Alternativ oder zusätzlich zu den Verfahrensschritten S112 und S113 kann ein weiterer nicht in Fig. 2 gezeigter Verfahrensschritt kennzeichnen, dass in Abhängigkeit von einer Vergrößerung des ersten Röntgenröhrenstromprofils durch den funktionellen Parameter des Patienten eine Verlängerung der zweiten Röntgenröhrenruhezeit erfolgt.

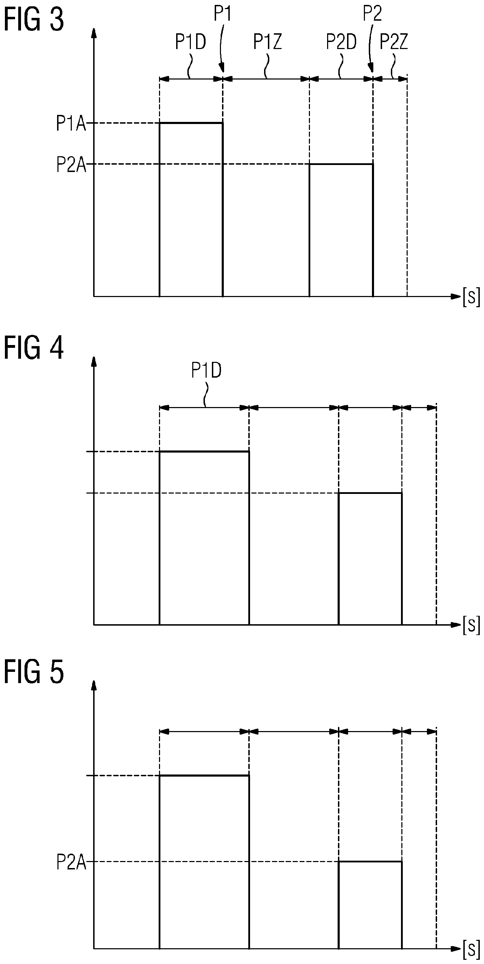

Fig. 3, Fig. 4 und Fig. 5 zeigen exemplarisch das Anpassen des zumindest einen Röntgenröhrenstromprofilparameters und des zweiten Röntgenröhrenstromprofils.

**Fig. 3** zeigt schematisch die mehreren Röntgenröhrenstromprofile P1, P2 vor einem Erfassen von ersten Rohdaten. Ein erstes Röntgenröhrenstromprofil P1 umfasst insbesondere eine erste Röntgenröhrenstromamplitude P1A, eine erste Röntgenröhrenstromdauer P1D und eine erste Röntgenröhrenruhezeit P1Z. Ein zweites Röntgenröhrenstromprofil P2 umfasst insbesondere eine zweite Röntgenröhrenstromamplitude P2A, eine zweite Röntgenröhrenstromdauer P2D und eine zweite Röntgenröhrenruhezeit P2Z. Aus Gründen der Übersichtlichkeit werden in Fig. 4 und Fig. 5 nur diejenigen Strukturen und Einheiten erneut gekennzeichnet, welche sich im Vergleich zur Fig. 3 wesentlich verändert haben.

**Fig. 4** zeigt schematisch die mehreren Röntgenröhrenstromprofile P1, P2 nach dem Erfassen der ersten Rohdaten und vor einem Anpassen des zweiten Röntgenröhrenstromprofils P2. Fig. 4 zeigt insbesondere, dass in diesem Ausführungsbeispiel die erste Röntgenröhrenstromdauer P1D gemäß einem funktionellen Parameter des Patienten während des Erfassens der ersten Rohdaten angepasst wird.

**Fig. 5** zeigt schematisch die mehreren Röntgenröhrenstromprofile P1, P2 nach dem Anpassen des zweiten Röntgenröhrenstromprofils P2. Fig. 5 zeigt insbesondere, dass in diesem Ausführungsbeispiel die zweite Röntgenröhrenstromamplitude P2A in Abhängigkeit von dem zumindest einen angepassten Röntgenröhrenstromprofilparameter, insbesondere in Abhängigkeit von der ersten Röntgenröhrenstromdauer P1D, angepasst wird.

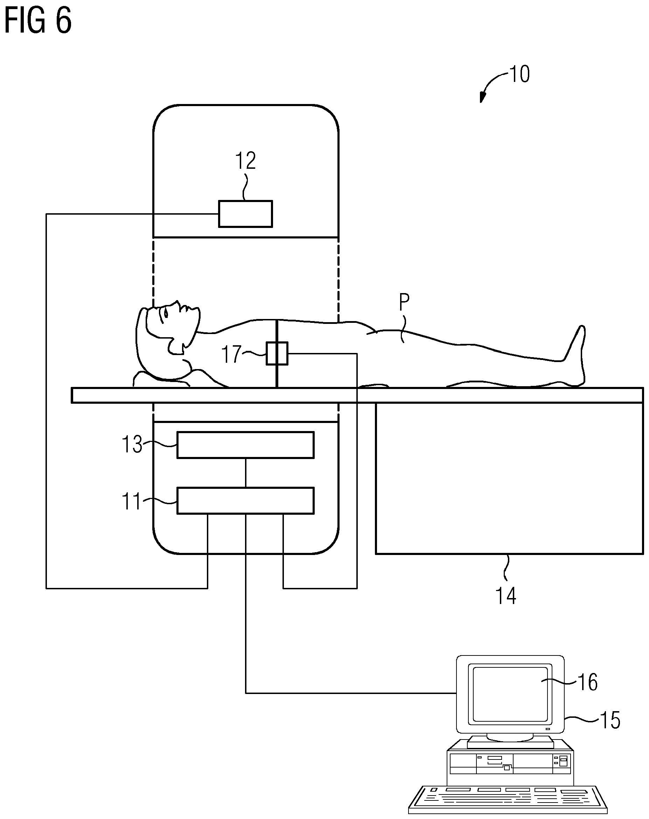

**Fig. 6** zeigt ein Computertomographiesystem 10, welches eine Steuereinheit 11 und eine Röntgenröhre 12 aufweist. Die Steuereinheit 11 kann in einer Recheneinheit des Computertomographiesystems 10 abgebildet sein. Das Computertomographiesystem ist in Fig. 6 in einem Querschnitt abgebildet.

Die Röntgenröhre 12 ist zu einer Emission von Röntgenstrahlung gemäß den mehreren Röntgenröhrenstromprofilen P1, P2 ausgebildet. Das Computertomographiesystem 10 weist typischerweise einen Röntgendetektor 13 auf, welcher zum Erfassen der Röntgenstrahlung nach dem Durchdringen des Patienten P ausgebildet ist, welcher auf einer Patientenliege 14 gelagert ist. Das Computertomographiesystem 10 weist eine Planungseinheit 15 mit einer Anzeigeeinheit 16 und Eingabemitteln auf. Der medizinische Bilddatensatz kann vorzugsweise auf der Anzeigeeinheit 16 bereitgestellt werden. In diesem Fall wird der medizinische Bilddatensatz also einem Nutzer vorzugsweise angezeigt. Das Computertomographiesystem 10 weist eine funktionelle Patientenüberwachungseinheit 17 auf, welche in diesem Ausführungsbeispiel einen Atemgurt aufweist.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren für ein Bereitstellen eines medizinischen Bilddatensatzes eines Patienten mittels einer Röntgenröhre eines Computertomographiesystems mit folgenden Schritten,
- Ermitteln von mehreren Röntgenröhrenstromprofilen der Röntgenröhre in einer Steuereinheit des Computertomographiesystems, wobei die mehreren Röntgenröhrenstromprofile ein erstes Röntgenröhrenstromprofil und ein zweites Röntgenröhrenstromprofil umfassen, ein Messprotokoll für eine bildgebende Messung in dem Computertomographiesystem bilden sowie eine Belastungsgrenze der Röntgenröhre unter Berücksichtigung eines funktionellen Referenzparameters einhalten,
- Erfassen von ersten Rohdaten des Patienten gemäß dem ersten Röntgenröhrenstromprofil mittels der Röntgenröhre, wobei ein funktioneller Parameter des Patienten erfasst wird und wobei zumindest ein Röntgenröhrenstromprofilparameter des ersten Röntgenröhrenstromprofils gemäß dem funktionellen Parameter während des Erfassens der ersten Rohdaten angepasst wird,
- Anpassen des zweiten Röntgenröhrenstromprofils in der Steuereinheit derart, dass das zweite Röntgenröhrenstromprofil in Abhängigkeit von dem zumindest einen angepassten Röntgenröhrenstromprofilparameter die Belastungsgrenze der Röntgenröhre einhält,
- Erfassen von zweiten Rohdaten des Patienten gemäß dem zweiten angepassten Röntgenröhrenstromprofil mittels der Röntgenröhre,
- Rekonstruieren des medizinischen Bilddatensatzes der bildgebenden Messung gemäß den ersten Rohdaten und den zweiten Rohdaten und
- Bereitstellen des medizinischen Bilddatensatzes des Patienten.

2. Verfahren nach Anspruch 1, wobei der funktionelle Parameter des Patienten eine Atmung und/oder eine Herzphase des Patienten beschreibt.

3. Verfahren nach einem der vorhergehenden Ansprüche, wobei sich das Messprotokoll für das Erfassen der ersten Rohdaten und das Messprotokoll für das Erfassen der zweiten Rohdaten in zumindest einem Parameter der folgenden Liste unterscheiden:
- in einer z-Position eines Messbereichs des Messprotokolls,
- in einer Phase innerhalb einer Periode einer physiologischen Funktion des Patienten.

4. Verfahren nach einem der vorhergehenden Ansprüche, wobei das Anpassen des zweiten Röntgenröhrenstromprofils unter Verwendung eines künstlichen neuronalen Netzes erfolgt.

5. Verfahren nach einem der vorhergehenden Ansprüche, wobei die Belastungsgrenze der Röntgenröhre einen Temperaturschwellwert der Röntgenröhre umfasst.

6. Verfahren nach einem der vorhergehenden Ansprüche, wobei der zumindest eine Röntgenröhrenstromprofilparameter eine erste Röntgenröhrenstromamplitude und/oder eine erste Röntgenröhrenstromdauer und/oder eine erste Röntgenröhrenruhezeit umfasst.

7. Verfahren nach einem der vorhergehenden Ansprüche, wobei das Anpassen des zweiten Röntgenröhrenstromprofils ein Anpassen einer zweiten Röntgenröhrenstromamplitude und/oder einer zweiten Röntgenröhrenstromdauer und/oder einer zweiten Röntgenröhrenruhezeit umfasst.

8. Verfahren nach einem der vorhergehenden Ansprüche, wobei in Abhängigkeit von einer Vergrößerung des ersten Röntgenröhrenstromprofils durch den funktionellen Parameter des Patienten eine Verkleinerung des zweiten Röntgenröhrenstromprofils zur Einhaltung der Belastungsgrenze der Röntgenröhre beim Anpassen erfolgt.

9. Verfahren nach Anspruch 8, wobei die zweite Röntgenröhrenstromamplitude verringert wird.

10. Verfahren nach einem der Ansprüche 8 bis 9, wobei die zweite Röntgenröhrenstromdauer verringert wird.

11. Verfahren nach einem der vorhergehenden Ansprüche, wobei in Abhängigkeit von einer Vergrößerung des ersten Röntgenröhrenstromprofils durch den funktionellen Parameter des Patienten eine Verlängerung der zweiten Röntgenröhrenruhezeit erfolgt.

12. Verfahren nach einem der vorhergehenden Ansprüche, wobei in Abhängigkeit von einer Verkleinerung des ersten Röntgenröhrenstromprofils durch den funktionellen Parameter des Patienten eine Vergrößerung des zweiten Röntgenröhrenstromprofils beim Anpassen erfolgt.

13. Verfahren nach Anspruch 12, wobei die zweite Röntgenröhrenstromamplitude und/oder die zweite Röntgenröhrenstromdauer vergrößert werden.

14. Computertomographiesystem, aufweisend
- eine Steuereinheit und
- eine Röntgenröhre,
wobei die Steuereinheit derart ausgebildet ist, dass sie ein Verfahren nach einem der vorhergehenden Ansprüche ausführt.

15. Computerprogrammprodukt, welches direkt in einen Speicher einer Recheneinheit ladbar ist, mit Programmcodemitteln, um ein Verfahren nach einem der Ansprüche 1 bis 13 auszuführen, wenn das Computerprogrammprodukt in der Recheneinheit ausgeführt wird.

## Claims

1. Method for provision of a medical image data set of a patient by means of an X-ray tube of a computed tomography system with the following steps,
- Determination of multiple X-ray tube current profiles of the X-ray tube in a control unit of the computed tomography system, wherein the multiple X-ray tube current profiles comprise a first X-ray tube current profile and a second X-ray tube current profile, form a measurement protocol for imaging measurement in the computed tomography system and satisfy a loading limit of the X-ray tube while taking into consideration a functional reference parameter,
- Collection of first raw data of the patient according to the first X-ray tube current profile by means of the X-ray tube, wherein a functional parameter of the patient is recorded and wherein at least one X-ray tube current profile parameter of the first X-ray tube current profile is adapted during the collection of the first raw data according to the functional parameter,
- Adaptation of the second X-ray tube current profile in the control unit in such a way that, as a function of the at least one adapted X-ray tube current profile parameter, the second X-ray tube current profile satisfies the loading limit of the X-ray tube,
- Collection by means of the X-ray tube of second raw data of the patient according to the second adapted X-ray tube current profile,
- Reconstruction of the medical image data set of the imaging measurement on the basis of the first raw data and the second raw data and
- Provision of the medical image data set of the patient.

2. Method according to claim 1, wherein the functional parameter of the patient describes a respiration and/or a cardiac phase of the patient.

3. Method according to one of the preceding claims, wherein the measurement protocol for the collection of the first raw data and the measurement protocol for the collection of the second raw data differ from one another in at least one parameter on the following list:
- in a z-position of a measurement range of the measurement protocol,
- in a phase within a period of a physiological function of the patient.

4. Method according to one of the preceding claims, wherein the adaptation of the second X-ray tube current profile is performed using an artificial neural network.

5. Method according to one of the preceding claims, wherein the loading limit of the X-ray tube includes a temperature threshold of the X-ray tube.

6. Method according to one of the preceding claims, wherein the at least one X-ray tube current profile parameter comprises a first X-ray tube current amplitude and/or a first X-ray tube current duration and/or a first X-ray tube idle period.

7. Method according to one of the preceding claims, wherein adaptation of the second X-ray tube current profile comprises adaptation of a second X-ray tube current amplitude and/or a second X-ray tube current duration and/or a second X-ray tube idle period.

8. Method according to one of the preceding claims, wherein a reduction in the second X-ray tube current profile occurs during adaptation, as a function of an extension of the first X-ray tube current profile as a result of the functional parameter of the patient, in order to satisfy the loading limit of the X-ray tube.

9. Method according to claim 8, wherein the second X-ray tube current amplitude is reduced.

10. Method according to one of claims 8 to 9, wherein the second X-ray tube current duration is reduced.

11. Method according to one of the preceding claims, wherein a lengthening of the X-ray tube idle time occurs as a function of an extension of the first X-ray tube current profile as a result of the functional parameter of the patient.

12. Method according to one of the preceding claims, wherein an extension in the second X-ray tube current profile occurs during adaptation as a function of a reduction of the first X-ray tube current profile as a result of the functional parameter of the patient.

13. Method according to claim 12, wherein the second X-ray tube current amplitude and/or the second X-ray tube current duration are increased.

14. Computed tomography system having
- a control unit and
- an X-ray tube,
wherein the control unit is configured in such a way that it executes a method according to one of the preceding claims.

15. Computer program product that is directly loadable onto a memory of a processing unit, with program code means for the purpose of executing a method according to one of claims 1 to 13 when the computer program product is executed in the processing unit.

## Revendications

1. Procédé pour se procurer un ensemble de données d'images médicales d'un patient, au moyen d'un tube à rayons X d'un système de tomodensitométrie à ordinateur, ayant les stades suivants,
- détermination de plusieurs profils de courant de tube à rayons X des tubes à rayons X dans une unité de commande du système de tomodensitométrie à ordinateur, les plusieurs profils de courant de tube à rayons X comprenant un premier profil de courant du tube à rayons X et un deuxième profil de courant du tube à rayons X formant un programme de mesure pour une mesure donnant une image dans le système de tomodensitométrie à ordinateur, ainsi que respectant une limite de charge des tubes à rayons X en tenant compte d'un paramètre de référence fonctionnel,
- relevé, au moyen des tubes à rayons X, de premières données brutes du patient suivant le premier profil de courant du tube à rayons X, un paramètre fonctionnel du patient étant relevé et au moins un paramètre de profil de courant du tube à rayons X du premier profil de courant du tube à rayons X étant adapté suivant le paramètre fonctionnel pendant le relevé des premières données brutes,
- adaptation du deuxième profil de courant du tube à rayons X dans l'unité de commande, de manière à ce que le deuxième profil de courant du tube à rayons X respecte, en fonction du au moins un paramètre de profil de courant du tube à rayons X adapté, la limite de charge des tubes à rayons X,
- relevé, au moyen du tube à rayons X, de deuxièmes données brutes du patient suivant le deuxième profil de courant du tube à rayons X adapté,
- reconstruction de l'ensemble de données d'image médicale de la mesure donnant une image suivant les premières données brutes et les deuxièmes données brutes et
- mise à dispositif de l'ensemble de données d'image médicale du patient.

2. Procédé suivant la revendication 1, dans lequel le paramètre fonctionnel du patient décrit une respiration et/ou une phase cardiaque du patient.

3. Procédé suivant l'une des revendications précédentes, dans lequel le programme de mesure pour le relevé des premières données brutes et le programme de mesure pour le relevé des deuxièmes données brutes se distinguent par au moins un paramètre de la liste suivante :
- par une position z d'une région de mesure du programme de mesure,
- par une phase dans une période d'une fonction physiologique du patient.

4. Procédé suivant l'une des revendications précédentes, dans lequel l'adaptation du deuxième profil de courant du tube à rayons X s'effectue en utilisant un réseau neuronal artificiel.

5. Procédé suivant l'une des revendications précédentes, dans lequel la limite de charge du tube à rayons X comprend une valeur de seuil de température du tube à rayons X.

6. Procédé suivant l'une des revendications précédentes, dans lequel le au moins un paramètre de profil de courant du tube à rayons X comprend une première amplitude de courant du tube à rayons X et/ou une première durée de courant du tube à rayons X et/ou un premier temps de repos du tube à rayons X.

7. Procédé suivant l'une des revendications précédentes, dans lequel l'adaptation du deuxième profil de courant du tube à rayons X comprend une adaptation d'une deuxième amplitude de courant du tube à rayons X et/ou d'une deuxième durée de courant du tube à rayons X et/ou d'un deuxième temps de repos du tube à rayons X.

8. Procédé suivant l'une des revendications précédentes, dans lequel, en fonction d'un agrandissement du premier profil de courant du tube à rayons X par le paramètre fonctionnel du patient, il se produit, lors de l'adaptation, un rapetissement du deuxième profil de courant du tube à rayons X pour respecter la limite de charge du tube à rayons X.

9. Procédé suivant la revendication 8, dans lequel on diminue la deuxième amplitude de courant du tube à rayons X.

10. Procédé suivant l'une des revendications 8 à 9, dans lequel on diminue la deuxième durée de courant du tube à rayons X.

11. Procédé suivant l'une des revendications précédentes, dans lequel, en fonction d'un agrandissement du premier profil de courant du tube à rayons X par le paramètre fonctionnel du patient, il se produit un allongement de la deuxième durée de repos du tube à rayons X.

12. Procédé suivant l'une des revendications précédentes, dans lequel, en fonction d'un rapetissement du premier profil de courant du tube à rayons X par le paramètre fonctionnel du patient, il se produit, lors de l'adaptation, un agrandissement du deuxième profil de courant du tube à rayons X.

13. Procédé suivant la revendication 12, dans lequel on agrandit la deuxième amplitude de courant du tube à rayons X et/ou la deuxième durée de courant du tube à rayons X.

14. Système de tomodensitométrie à ordinateur, comportant
- une unité de commande et
- un tube à rayons X,
dans lequel l'unité de commande est constituée de manière à réalise un procédé suivant l'une des revendications précédentes.

15. Produit de programme d'ordinateur, qui peut être chargé directement dans une mémoire d'une unité informatique, comprenant des moyens de code de programme pour exécuter un procédé suivant l'une des revendications 1 à 13, lorsque le produit de programme d'ordinateur est réalisé dans l'unité informatique.
